# FASCICULE DE BREVET EUROPEEN

(11) **EP 4 454 689 B1**
(45) Date de publication et mention de la délivrance du brevet: **17.12.2025**
(21) Numéro de dépôt: 24166452.3
(22) Date de dépôt: 26.03.2024
(51) Int. Cl.: A61M 16/00

(54) **APPAREIL D ASSISTANCE RESPIRATOIRE À PRESSION POSITIVE CONTINUE**
KONTINUIERLICHES POSITIVDRUCK-ATEMUNTERSTÜTZUNGSGERÄT
CONTINUOUS POSITIVE PRESSURE BREATHING ASSISTANCE APPARATUS

(30) Priorité: 27.04.2023 FR 2304287
(43) Date de publication de la demande: 30.10.2024
(73) Titulaire: SEFAM, 92200 Neuilly sur Seine (FR)
(72) Inventeur: POIROT, Bertrand, 54110 VARANGEVILLE (FR); CLERC, Nicolas, 54000 NANCY (FR); SERIN, Adrien, 54130 SAINT-MAX (FR); LAMY, Jean-Sébastien, 54710 LUDRES (FR)
(74) Mandataire: Littolff, Denis

(56) Documents cités:
- WO-A1-2005/097244
- WO-A1-2013/020167
- WO-A1-2021/135615
- WO-A1-2022/094655
- WO-A1-2022/141536
- WO-A1-2023/043857
- WO-A1-2023/283001
- WO-A1-99/22794
- US-A1- 2016 199 605

## Description

La présente invention relève du domaine des appareils d'assistance respiratoire aptes à exercer une ventilation à pression positive continue (PPC) pour traiter les troubles respiratoires liés au sommeil se manifestant sous forme d'apnées et/ou d'hypopnées obstructives. L'invention a de fait vocation à traiter les effets de ce qu'il est convenu d'appeler les SAHOS, pour Syndrome d'Apnée ou Hypopnée Obstructive du Sommeil, spécifiquement dans les phases d'endormissement puis de sommeil des personnes qui en sont affectées. Il s'agit en fait de pauses involontaires de la respiration résultant d'une fermeture temporaire et plus ou moins complète des voies respiratoires, par exemple causées par un relâchement de la langue et des muscles de la gorge. On sait à présent que ces troubles présentent un lien étroit avec la prévalence d'autres pathologies pour lesquelles ils sont un facteur d'augmentation du risque d'apparition, comme l'hypertension et les maladies cardiovasculaires.

Pour pallier les inconvénients et risques nés de ces arrêts temporaires du système respiratoire, des appareils de ventilation tels que celui de l'invention sont utilisés, aux fins d'empêcher ou de remédier à l'obstruction desdites voies en y créant une pression positive qui les maintient artificiellement ouvertes dès qu'un déficit de pression et/ou de débit est détecté. Ces appareils dits à pression positive continue (PPC) sont des dispositifs électriques basés sur un compresseur d'air, un air pressurisé à un niveau prédéterminé étant nécessaire pour désobstruer les voies respiratoires dès lors qu'une fermeture est mesurée.

Or, le problème qui se pose d'emblée lors de la conception d'un appareil à PPC est le niveau sonore qu'il produit au cours de son fonctionnement. On comprend bien que l'usage de ces appareils se faisant de nuit, dans un processus d'accompagnement du sommeil, la notion de bruit de fonctionnement s'avère antinomique. L'existence d'un tel bruit peut d'ailleurs constituer un facteur de perturbation sur plusieurs niveaux : outre la gêne induite au cours du traitement, lorsqu'une personne l'accepte et utilise l'appareil, la nuisance sonore peut être dissuasive en amont du traitement, et conduire à le refuser.

En pratique, pour rendre l'appareil - et donc le traitement - plus acceptable, il est souhaitable de réduire autant que possible le niveau sonore global de l'appareil, afin de ne pas perturber les nuits de sommeil des utilisateurs, et éventuellement de convaincre les réticents que la nuisance sonore est maintenue à un niveau qui ne les gênera pas.

Un des moyens d'atténuation du bruit bien connu consiste en l'utilisation de mousses absorbantes, positionnées à des endroits appropriés de la machine. Cela peut être à l'écart du flux d'air généré dans l'appareil, par exemple dans les semelles placées sous l'appareil, pour atténuer les vibrations. La mousse peut également être disposée entre les organes bruyants internes et des capots externes, etc. Les solutions utilisées peuvent aussi être prévues dans le flux d'air, en réalisant par exemple tout ou partie des parois guidant ledit flux avec de la mousse. Cette disposition s'avère cependant problématique car les mousses présentent une résistivité aéraulique. Une partie de l'énergie cinétique du flux d'air se disperse dans la mousse et il en résulte, à géométrie de conduite équivalente, une perte de performance aéraulique. Un second problème concerne la sécurité du patient. Les mousses absorbantes peuvent en effet, selon leur matériau et les conditions d'utilisation, se désagréger après un certain temps et/ou à l'exposition prolongée à l'humidité. Dans un tel cas, il existe un réel risque de détachement, puis de déplacement des éléments détachés avec le flux d'air de la machine, et ensuite d'ingestion de ces particules par le patient. Il y a de plus un risque d'altération chimique du matériau (par hydrolyse notamment).

Or, les appareils médicaux pour le traitement de l'apnée du sommeil sont soumis à des exigences réglementaires strictes, avec des standards normatifs qui évoluent régulièrement. Il est arrivé qu'un fabricant soit contraint à un rappel de parc à la suite de plaintes : les mousses se dégradaient et des particules étaient ingérées par les patients, dont plusieurs se sont ensuite plaints de douleurs et maux de tête possiblement liés à cette ingestion. Il est en effet à présent connu que certains matériaux des mousses se dégradent par hydrolyse et génèrent des corps organiques volatiles (COV) incompatibles avec les standards de sécurité, la possible altération chimique de ces particules après hydrolyse pouvant les rendre potentiellement toxiques. Cela dépend en fait du matériau de la mousse choisie pour équiper l'appareil. Il est à noter que les mousses sont de plus en général difficilement nettoyables ou désinfectables.

Un autre problème technique rencontré, bien que moindre, relève de l'industrialisation et a trait au procédé d'assemblage du produit fini. Les mousses absorbantes sont généralement molles et déformables. Lors du montage de l'appareil par l'opérateur de production, il est possible qu'une mousse soit, selon la complexité du montage, mal positionnée ou déformée. Cela peut avoir pour conséquence de dégrader l'efficacité phonique et/ou aéraulique du conduit.

Le moyen le plus efficace de réduire le niveau sonore final d'un produit est de réduire l'émission sonore des éléments sources de nuisance. Les problèmes de niveau sonore dans des dispositifs électromécaniques organisant une compression gazeuse sont d'origines diverses, et les solutions pour y répondre ont chacune leurs limites. Dans les appareils à PPC, la principale source de bruit est le compresseur, et la première action envisageable est de limiter les transmissions de vibrations du compresseur vers le reste de l'appareil, mais cela ne concerne alors que la partie « vibratoire » du niveau sonore, qui pourra être amortie par les moyens choisis. Les autres causes de bruit trouvent leur source en général dans l'aéraulique : il s'agit de tous les frottements et percussions des flux gazeux qui parcourent la machine, et qui ne sont ne sont pas (ou très peu) réduits par les moyens classiques évoqués.

Pour réduire les émissions sonores qui proviennent de l'aéraulique, il est dès lors logique de chercher à diminuer les frottements, les recirculations, les turbulences et les changements de direction des flux d'air dans les tubes, conduits, canalisations. On cherche par exemple à adoucir les angles et les courbures sur le parcours de l'air dans l'appareil. Ces solutions techniques ne sont cependant également que partiellement efficaces, et on ne peut pas faire l'économie d'une réflexion sur la partie « vibratoire ». WO2023/043857 A1 propose l'utilisation de canaux en forme de nids d'abeilles (13, fig.4) au niveau du canal d'entrée d'un appareil d'assistance respiratoire de type à pression positive continue pour le traitement des troubles respiratoires du sommeil.

La présente invention propose une solution qui s'affranchit des conceptions traditionnelles basées sur l'utilisation de mousses absorbantes faisant office d'amortisseur acoustique, et est au contraire basée sur l'emploi d'élément rigides judicieusement positionnés et dimensionnés pour remplacer efficacement lesdites mousses. La conception de ces éléments rigides et le choix de leur emplacement d'implantation a un double objectif. Ils produisent d'abord une atténuation phonique, par leur effet direct sur la propagation de l'onde sonore dans l'air. Ils visent ensuite à une atténuation mécanique, puisqu'ils sont prévus pour renforcer la rigidité des pièces mécaniques dans lesquelles ils sont implémentés, qui deviennent de ce fait moins susceptibles de vibrer ou de se déformer, et donc de générer du bruit.

A ces effets, et pour d'autres résultats qui seront décrits plus en détail dans la suite, l'appareil d'assistance respiratoire de type à pression positive continue pour le traitement des troubles respiratoires du sommeil de l'invention comporte classiquement un conduit d'entrée d'air acheminant un flux d'air entrant vers un compresseur, le flux d'air comprimé en sortie du compresseur étant relié à un conduit de sortie d'air pressurisé à destination d'un masque prévu pour s'appliquer aux voies respiratoires d'un patient. A partir de ce schéma de base traditionnel, la conception de l'invention propose de nombreux aménagements apportant localement des solutions au problème global de la nuisance sonore.

A titre principal, selon l'invention, l'appareil à PPC est d'abord tel que le conduit d'entrée d'air débouche dans un compartiment en amont - selon le sens de la circulation du flux d'air - de l'entrée du compresseur, et présente à ce niveau des moyens de guidage du flux d'air d'abord vers une paroi de fond dudit compartiment orientée sensiblement perpendiculairement à la direction d'aspiration de l'air dans le compresseur, au moins ladite paroi de fond étant au moins partiellement recouverte d'une structure à motifs alvéolaires rigide et indéformable.

En somme, on a placé dans ce compartiment en amont du compresseur des motifs alvéolaires particuliers, présentant une géométrie prédéterminée et qui tapissent une paroi du compartiment. Ils sont positionnés dans l'appareil de sorte qu'ils y sont inamovibles, le matériau solide utilisé les rendant au surplus non-dégradables dans la durée. La structure à motifs alvéolaires est en l'espèce orientée sensiblement parallèlement au flux de l'air tel qu'il est guidé en sortie de cette portion du conduit d'entrée débouchant dans le compartiment, et le flux gazeux traverse donc frontalement ces motifs en se dispersant sur la surface de la paroi de fond du compartiment. D'un point de vue aéraulique, les motifs alvéolaires permettent de linéariser le flux d'air, et donc le rendre moins turbulent. Or, ce sont les turbulences dans un flux d'air qui sont génératrices de bruit.

Plus précisément, selon une configuration propre à l'appareil de l'invention, le conduit d'entrée d'air comporte, à l'entrée dudit compartiment, une rampe suivie d'une portion spiralée se développant dans les motifs alvéolaires de la paroi de fond, ladite portion spiralée présentant un sabot terminal creux arrondi, centré dans ladite paroi de fond et localisé au droit de l'axe du compresseur. Le flux d'air entrant est par conséquent dirigé vers le fond du compartiment, selon une direction d'arrivée dont on a vu qu'elle est globalement parallèle à lui, puis renvoyé dans une direction perpendiculaire vers l'entrée du compresseur. Cette portion du conduit d'entrée permet de canaliser le flux amont sous le compresseur de sorte qu'il y ait le moins de perte de charge et le moins de turbulences possibles. L'orientation (ici centripète selon le sens horaire) de la spirale permet d'amorcer une giration du flux avant qu'il n'accède au compresseur, et qu'il subisse ensuite une giration encore plus forte dans le même sens. Cela contribue aussi à une meilleure canalisation du flux.

La structure alvéolée du fond est en fait positionnée en proximité directe de la zone d'aspiration du compresseur, là où le flux d'air arrivant est le plus turbulent et où certaines fréquences du son émis par le compresseur sont générées. Pour améliorer encore le contrôle de la linéarité du flux entrant dans le compartiment, la portion spiralée est munie, en amont du sabot arrondi, d'une nervure séparatrice qui a pour fonction d'abord de mieux canaliser le flux d'air puis de diminuer les turbulences. L'évidement du sabot terminal creux est par ailleurs fermé par un guide périphérique incurvé qui aide à rediriger au moins une partie du flux vers la turbine.

Par ailleurs, de préférence, la rampe et la portion spiralée constituent la face supérieure d'un bloc dont le socle comporte une structure à motifs alvéolaires, qui permet, outre le renforcement de la rigidité dudit bloc, également de réduire le niveau sonore, la rigidification mécanique aidant à supprimer ou à réduire certaines vibrations. Ce bloc est d'ailleurs préférentiellement fabriqué distinct du compartiment et solidarisé notamment à son fond alvéolé. Le reste du fond alvéolé du compartiment peut soit être une pièce distincte reposant sur le fond du compartiment, soit être réalisé au cours de l'injection du reste de la demi-coque du châssis, et être par conséquent solidaire dudit fond.

Selon une configuration possible de l'invention, l'appareil à PPC comporte un espace tampon prévu en amont du compresseur, entre d'une part une paroi de séparation séparant le compartiment du compresseur, ladite paroi étant munie d'une ouverture placée au droit du sabot terminal de la portion spiralée, et d'autre part ledit compresseur. Il s'agit en fait d'un volume « tampon » qui est laissé au flux d'air, entre le moment où il sort du compartiment et le moment où il entre dans le compresseur. Cet espace participe à la diminution du niveau sonore.

Par ailleurs, en amont de la rampe, le conduit d'entrée d'air de l'appareil peut comporter deux tronçons d'allure parallèle superposés, un premier tronçon relié à l'entrée d'air de l'appareil et un second tronçon débouchant dans la rampe, lesdits tronçons étant reliés par une portion arrondie en U. Les deux tronçons sont par exemple tels que le second est placé au-dessus du premier et comporte l'accès à la rampe qui descend vers le compartiment, ce dernier étant dès lors situé au même niveau « inférieur » que le premier tronçon.

Le premier tronçon peut comporter, sur une longueur suffisante, un profil linéarisateur, constitué d'un motif alvéolaire orienté dans le sens de passage du flux. Cette structure est positionnée en aval de l'entrée d'air de l'appareil, d'où le flux peut potentiellement sortir dans un régime turbulent. En effet, les variations de sections transversales, les changements d'orientations, et les éventuels éléments singuliers (grilles, filtres, formes environnantes au conduit) sont autant d'obstacles à la laminarité du flux.

De préférence, le second tronçon peut comporter une sonde Pitot dont une semelle reposant sur une paroi de séparation des tronçons superposés présente une structure à motifs alvéolaires qui rigidifie cette partie de l'appareil et l'empêche notamment de vibrer. Cette structure à motifs alvéolaires permet par ailleurs de limiter la transmission du son issu du premier tronçon vers le second, et inversement.

A une des deux extrémité du circuit aéraulique de l'appareil PPC, le conduit de sortie en aval du compresseur peut être équipé d'une pièce terminale d'atténuation des vibrations et du bruit comportant une chicane de circulation de l'air sous forme d'une tubulure présentant au moins un virage et se développant dans un plan frontal d'allure perpendiculaire à la direction du flux d'air dans la portion finale du conduit de sortie, les bouches d'entrée et de sortie de ladite tubulure étant sensiblement parallèle à ladite direction.

Les vibrations de l'appareil de l'invention ainsi que le bruit du compresseur peuvent en réalité être perceptibles jusqu'au masque porté par le patient, d'où l'importance de les supprimer ou à tout le moins de les réduire considérablement, par les moyens et caractéristiques déjà décrites et à venir.

Plus précisément, la tubulure de cette pièce terminale d'atténuation des vibrations et du bruit est constituée à partir de deux parties de ladite pièce fixées l'une à l'autre, un socle et une façade orientés selon ledit plan frontal, et en ce qu'au moins la face du socle sur laquelle la façade est fixée est recouverte d'une structure à motifs alvéolaires. C'est la face orientée vers l'extérieur du socle qui est revêtue de ces motifs, qui permettent d'amortir encore un peu plus le niveau sonore.

A l'autre bout du circuit aéraulique, le conduit d'entrée d'air est fermé, à son entrée, par un capot porte-filtre muni d'une grille décalée par rapport à l'axe du conduit d'entrée, ledit capot étant solidarisé à un pavillon s'évasant à partir du conduit d'entrée et acheminant l'air depuis la grille audit conduit. Le flux d'air entre donc dans l'appareil en traversant la grille - derrière laquelle est placé un ou plusieurs filtres - de préférence orientée perpendiculairement à l'axe du conduit de sorte que le flux entrant y est parallèle audit axe, puis est entraîné en direction du conduit d'entrée, ce qui l'oblige à changer de direction et à prendre une orientation normale à la première orientation lors du franchissement de la grille. Le flux d'air connaît ensuite un second virage opposé, de sorte à revenir dans la direction du conduit d'entrée, qui est en l'occurrence et de préférence le premier tronçon du conduit d'entrée. La position en hauteur de l'entrée d'air par la grille de l'appareil a fait l'objet d'itérations. En effet, si elle est située trop bas par rapport au conduit d'entrée, les ondes sonores émises depuis cette zone peuvent se réverbérer sur la surface d'appui de l'appareil (par exemple la table de chevet ou le sol sur lequel repose la machine en fonctionnement), ce qui constitue un phénomène indésirable.

Selon une possibilité, des parois latérales d'allure perpendiculaire à la surface de la grille au niveau du capot ou du pavillon comportent des lames obliques inclinées, dépassant desdites parois latérales en direction du centre de la grille, formant un dispositif surnommé « piège à poisson » disposé pour empêcher les ondes sonores de remonter vers la grille d'entrée du châssis du compresseur. Par ailleurs, le capot muni de la grille présente également, sur sa face interne en face du conduit d'entrée, une structure à motifs alvéolaires. Ce n'est donc pas une paroi pleine et plate qui est positionnée face au départ du conduit, qui n'aurait aucune incidence en termes d'atténuation des turbulences présentes dans le flux d'air entrant.

En pratique, de préférence, chaque structure à motifs alvéolaires comporte des alvéoles hexagonales, ce qui en fait une structure en nid d'abeilles. Le dimensionnement desdits motifs en nid d'abeille est subordonné au fait que les épaisseurs des parois des motifs sont de l'ordre de 1,5 mm maximum. Les espaces vides peuvent présenter des dimensions de 3 à 4 mm entre parois. En pratique, les parois des motifs doivent être suffisamment épaisses et denses pour rigidifier la structure sur laquelle elles sont positionnées, sans pour autant générer des défauts de plasturgie (retassures notamment).

Selon une vue plus globale de la constitution de l'invention, le compresseur est fixé dans un châssis comportant deux demi-coques superposées séparées par un joint médian souple, les conduits d'entrée et de sortie étant au moins partiellement conformés dans ledit châssis. Plus précisément, en lien avec la configuration expliquée auparavant, une sonde Pitot et un bloc à rampe et portion spiralée du conduit d'entrée sont notamment immobilisés dans et par ledit châssis, entre le joint médian souple et les demi-coques, le joint médian souple formant une paroi de séparation d'une part entre deux tronçons superposés du conduit d'entrée et d'autre part entre le compresseur et un compartiment au débouché de la rampe, ledit joint comportant une ouverture placée au-dessus de l'extrémité de ladite portion spiralée et sous l'entrée du compresseur.

En d'autres termes, lorsque l'appareil de l'invention est posé sur un plan horizontal, cette configuration comporte en pratique :
- des moyens de maintien d'un ou plusieurs filtres formant une chicane à l'entrée suivis de deux tronçons de conduites horizontales placés l'un au-dessus de l'autre,
- puis une voie de descente incurvée vers le fond tapissé de motifs alvéolaires d'un compartiment,
- surmonté d'un compresseur dont le conduit de sortie est légèrement incurvé,
- et qui débouche dans une pièce terminale de réduction de bruit présentant également une chicane.

Il s'agit d'une configuration présentant une architecture strictement propre à l'invention. La plupart des caractéristiques techniques mentionnées ci-dessus, dont les configurations respectives des différentes parties, et l'existence de différentes zones à motifs alvéolaires, mettent en œuvre les deux fonctions déjà évoquées :
- Une atténuation phonique, par leur effet sur la propagation de l'onde sonore dans l'air : l'onde vient par exemple se réfléchir dans les parois à motifs alvéolaires, ce qui peut entraîner une annulation (par opposition de phase) ou une atténuation locale de la propagation du son, et
- Une atténuation mécanique, puisque les structures hexagonales des motifs alvéolaires viennent renforcer la rigidité des pièces mécaniques qui les logent, lesquelles cessent de vibrer ou de se déformer, ou réduisent ces phénomènes qui génèrent du bruit.

D'autres buts et avantages de la présente invention apparaîtront au cours de la description qui va suivre, se rapportant à une configuration qui n'est donnée qu'à titre d'exemple indicatif. La compréhension de cette description sera en particulier facilitée par référence aux dessins joints en annexe et dans lesquels :
La figure 1 montre une vue en perspective d'un châssis d'appareil à pression positive continue selon la présente invention ;
La figure 2 en représente une vue en élévation du côté de l'entrée d'air ;
La figure 3 est une vue en coupe de la partie d'entrée du circuit aéraulique ;
La figure 4 montre en vue en perspective du châssis du compresseur, sans la demi-coque supérieure du châssis et sans le compresseur ;
La figure 5a et la figure 5b représentent une sonde Pitot modifiée selon l'invention, respectivement en vue perspective et selon une coupe transversale ;
La figure 6 illustre en vue perspective la configuration du joint souple médian ;
La figure 7 montre une vue en coupe selon un plan perpendiculaire au plan de section de la figure 3, dans la partie du conduit d'entrée comportant une rampe descendante ;
La figure 8 est une vue en perspective de la demi-coque inférieure du châssis avec les divers éléments qui y reposent ;
La figure 9 montre une vue en coupe selon un plan perpendiculaire au plan de section de la figure 7 dans le compartiment dans lequel débouche la rampe descendante, en l'absence de la demi-coque supérieure ; et
La figure 10a et la figure 10b montrent en vue perspective la pièce terminale d'atténuation des vibrations et du bruit positionnée à l'issue du conduit de sortie de l'air pressurisé.

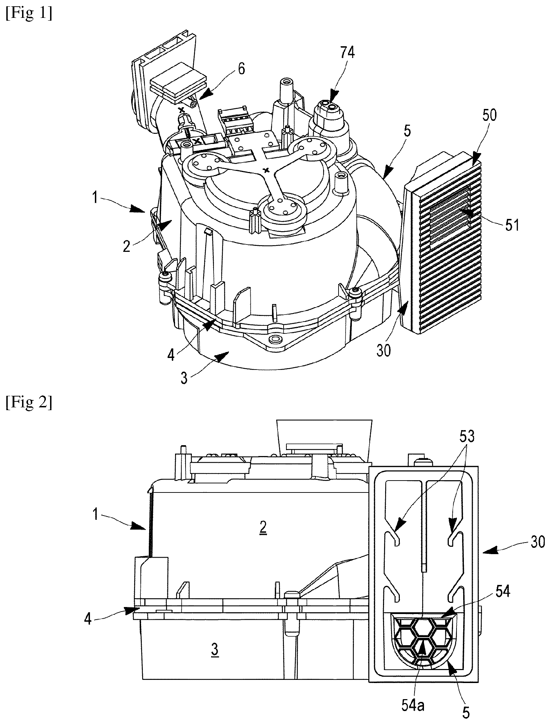

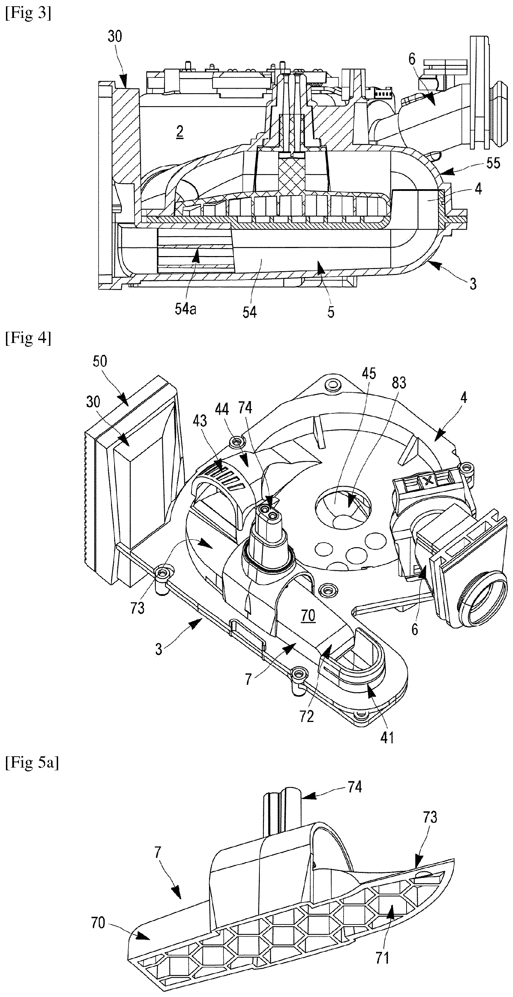

En référence à la figure 1, l'appareil à pression positive continue de l'invention comporte un châssis 1 composé de deux demi-coques respectivement supérieure 2 et inférieure 3, séparées par un joint souple 4. Un capot porte-filtre 50 d'entrée comportant une paroi dotée d'une grille 51 est disposé à l'extrémité du conduit d'entrée 5 décrit ultérieurement, fixé à une bouche s'évasant en un pavillon 30 fermé par ledit capot 50, ledit pavillon 30 étant partie intégrante de la demi-coque inférieure 3. A l'autre extrémité du circuit aéraulique interne à l'appareil, un conduit de sortie 6 sort du châssis 1 à l'opposé de la pièce filtre 50 d'entrée. Le capot 50 muni de la grille 51 comporte par ailleurs sur une partie de sa face intérieure une structure à motifs alvéolaires, en face de l'extrémité du conduit d'entrée 5. Les alvéoles forment un relief de rigidification situé en face du départ d'un premier tronçon 54 du conduit d'entrée (voir en figure 3). Les parois latérales d'allure perpendiculaire à la grille 51 de l'ensemble capot porte-filtre 50 / pavillon 30 comportent des lames obliques 53 inclinées vers le bas. Elles sont prévues pour « piéger » le bruit en l'empêchant de remonter vers la grille 51 et d'y ressortir par cette voie. Plus précisément, ces lames 53 dépassent symétriquement de chaque paroi latérale, et s'orientent obliquement simultanément vers le bas du capot 50 et en direction de la paroi latérale opposée. Un ou plusieurs filtres (non représentés) sont par ailleurs positionnés entre ces lames 53 et la grille 51, au-dessus de l'entrée du conduit 5.

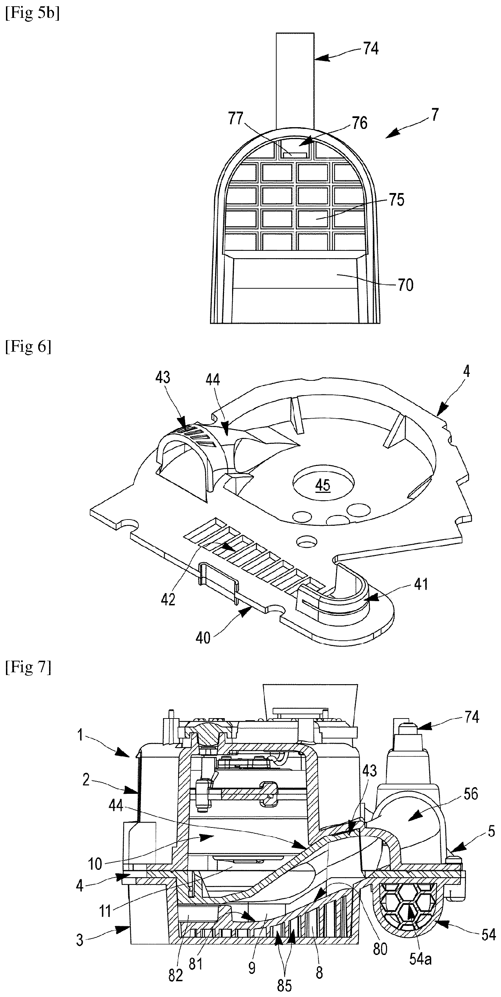

Le flux d'air parcourant cette partie d'entrée du circuit aéraulique change de direction après passage de la grille 51, d'horizontale à verticale, puis change à nouveau de direction au niveau de la structure alvéolaire placée au bas du capot 50, qui atténue l'effet des turbulences du flux, pour s'engager horizontalement dans le premier tronçon 54 du conduit d'entrée 5 et traverser le profil linéarisateur 54a, dont la section présente un motif alvéolaire (les mentions d'horizontalité et de verticalité se réfèrent aux figures, et ne doivent pas être entendues dans l'absolu). Une portion en U 55 au bout dudit premier tronçon 54 entraîne le flux en sens inverse dans un second tronçon 56 situé au-dessus du premier. Les deux tronçons 54, 56 superposés sont en pratiques séparés l'un de l'autre d'une part par une partie 40 du joint souple 4 (voir en figure 6), qui comporte à cet endroit un muret 41 arrondi participant au guidage du demi-tour du flux dans la portion en U 55 disposée entre les deux tronçon 54, 56, et d'autre part par la semelle 70 d'une sonde Pitot 7 apparaissant en figures 5a et 5b, munie d'une grille intérieure 75 normale au sens du flux d'air (visible en figure 5b). Le but de cette pièce est de créer une légère différence de pression entre l'amont et l'aval de la grille. Cette différence de pression est proportionnelle au débit traversant. Un capteur (placé sur le dessus de la cheminée 74 dépassant de la demi-coque supérieure 2 du châssis 1 vers le haut) interprète ce delta de pression en une valeur proportionnelle de débit.

Les deux tronçons 54 et 56 sont ainsi formés par un sous-ensemble d'un nombre minime de pièces (ici 4). La forme de la section transversale de ces tronçons, ainsi que celle de la portion 55, est en U, dénommée « semi-oblongue ». C'est particulièrement visible en figure 7, montrant bien les deux tronçons 54, 56 superposés, disposés en U inversés puisque le U du tronçon inférieur 54 est à l'endroit alors que le U du tronçon supérieur 56 est retourné. Il s'agit de la résultante d'un compromis entre, d'une part, le nombre, la disposition, les fonctions mécaniques des pièces formant ce profil, et, d'autre part, les contraintes propres à la plasturgie de chacune des pièces (épaisseurs de matière constantes, dépouilles, simplicité de conception etc.). Pour linéariser au mieux un flux d'air, la section de passage idéale a une forme circulaire. Le compromis ici présenté a donc consisté à approcher le mieux possible cette forme tout en conservant un plancher (ou un plafond selon le conduit concerné) plat pour simplifier la plasturgie et l'assemblage des éléments. Le choix de cette géométrie s'appuie aussi sur des simulations numériques mettant en évidence sa pertinence.

La sonde Pitot 7 comporte une section de passage du flux d'air similaire au reste des conduits en amont et aval de ladite sonde 7 (cette section est donc semi-oblongue). Elle doit assurer la fonction d'organe déprimogène décrite précédemment sans pour autant générer un obstacle au flux traversant, au point de créer des pertes de charges ou des turbulences indésirables. La section de passage de la sonde Pitot 7 comporte deux zones fonctionnelles principales : la grille 75 avec des sections rectangulaires pour la plupart traversantes qui contribuent à linéariser le flux et une section obstruée qui forme un obstacle 76. Celui-ci est plus précisément constitué d'une paroi obstructive 76 et d'une ouverture 77 placé dans la partie basse de cette paroi 76 obstruée. L'ouverture 77 est particulièrement dimensionnée et particulièrement positionnée sur le flux d'air. C'est en amont et aval de l'obstacle 76 que les mesures de pressions proportionnelles au débit traversant sont réalisées. Chacune des deux ouvertures supérieures de la cheminée 74 débouche, pour l'une, juste un peu en amont et pour l'autre, juste un peu en aval de cette paroi obstructive 76. Le dimensionnement de ces zones fonctionnelles a fait l'objet d'essais de performances aérauliques, phoniques et de simulations numériques itératives.

La semelle 70 de la sonde Pitot 7 comporte également une structure à motifs alvéolaires 71 qui repose sur une surface de la partie 40 du joint 4 munie d'un motif particulier 42 à évidements rectangulaires successifs (voir en particulier en figure 6). Au-dessus de la structure alvéolée 71, la surface supérieure de la semelle 70 est configurée pour permettre un lissage du guidage du flux d'air, prévu notamment pour empêcher les turbulences dans les courbes du trajet emprunté par le flux d'air. Ainsi, l'extrémité d'entrée 72 de la semelle 70 est recourbée et forme une surface de guidage intérieure de la portion en U.

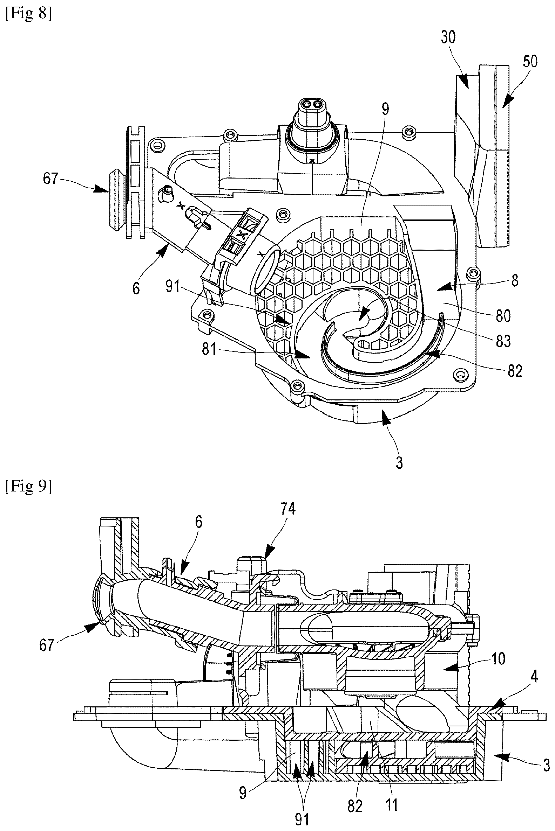

En sortie de la sonde Pitot 7, l'extrémité de sortie 73 de la semelle 70 présente une forme d'amorçage d'un virage vers la partie suivante du conduit d'entrée 5, comportant une rampe 80 descendant vers un compartiment inférieur 9. Celui-ci est situé, dans le châssis 1, au même niveau que le premier tronçon 54 du conduit d'entré. A partir de ladite rampe 80, la paroi qui enveloppe et délimite le conduit d'entrée 5 ne relève plus des demi-coques 2, 3, comme c'était au moins partiellement le cas jusqu'alors. Une voûte 43 du joint 4 suivie d'un pan incliné 44 de ce même joint 4 forment la partie supérieure du conduit, dont la piste inférieure relève d'un bloc 8 (voir en particulier en figure 8) comportant la rampe 80 et une portion spiralée 81 se terminant dans le fond du compartiment 9. Le socle de ce bloc 8 est également muni d'une structure à motifs alvéolaires 85. La portion spiralée 81 est équipée d'une nervure séparatrice 82 de fluidification de l'air et d'atténuation des turbulences lors du guidage du flux d'air dans la spirale.

Le fond du compartiment 9 comprend aussi une structure à motifs alvéolaires 91. Après le dernier virage de la portion spiralée 81, tout le fond est rempli par des motifs 91 en nid d'abeille, pour lesquels la dimension des alvéoles a fait l'objet d'itérations jusqu'à trouver la forme satisfaisante, en l'occurrence hexagonale.

Cette structure à motifs alvéolaires 91 placée dans le fond du compartiment 9 est particulièrement importante pour la résolution des problèmes sonores, du fait de son positionnement à proximité immédiate de la zone d'aspiration du compresseur 10, là où le flux d'air est le plus turbulent et où certaines fréquences du son émis par le compresseur 10 sont générées. Il est à noter que cette structure 91 peut être fabriquée de manière indépendante, c'est-à-dire qu'il s'agit d'un composant à part placé dans le fond du compartiment 9 de la demi-coque inférieure 3 du châssis 1 ou, alternativement, injectée avec le reste de la demi-coque 3 et donc d'emblée solidaire du châssis 1.

Le sabot terminal 83 creux et arrondi de la portion spiralée 81 présente un guide périphérique sous forme d'un versant qui s'incurve depuis le fond de l'évidement central du sabot 83 situé dans le prolongement de la piste spiralée 81 vers un rebord situé au niveau de la surface supérieure des motifs alvéolaires 91. Ce sabot terminal 83 est situé sous une ouverture 45 circulaire pratiquée dans une zone centrale du joint souple 4 (voir en particulier en figure 6). Lorsque l'air arrive au bout de la nervure 82 de la piste de la portion spiralée 81, au niveau du sabot terminal 83, il est juste en dessous du compresseur 10, prêt à être aspiré.

Comme cela apparaît particulièrement clairement en figure 9, le compresseur 10 est située au-dessus du compartiment 9, dont il est séparé par le joint souple 4. Il ne repose pas sur ledit joint 4, un espace tampon 11 ayant été ménagé pour le flux d'air entrant, situé entre l'endroit de sa sortie du compartiment inférieur 9, et l'endroit où il entre dans la turbine. L'existence de cet espace 11 est très importante pour la diminution du niveau sonore de l'appareil de l'invention. Le compresseur 10 est sensiblement centré au-dessus de l'ouverture 45.

Une fois compressé dans le compresseur 10, le flux d'air est envoyé à travers un conduit de sortie 6 (voir notamment en figure 9) avant de sortir du châssis 1. Ses dimensions et ses courbures ont été optimisées, en s'appuyant sur des simulations numériques, pour y atténuer autant que possible le bruit et les vibrations.

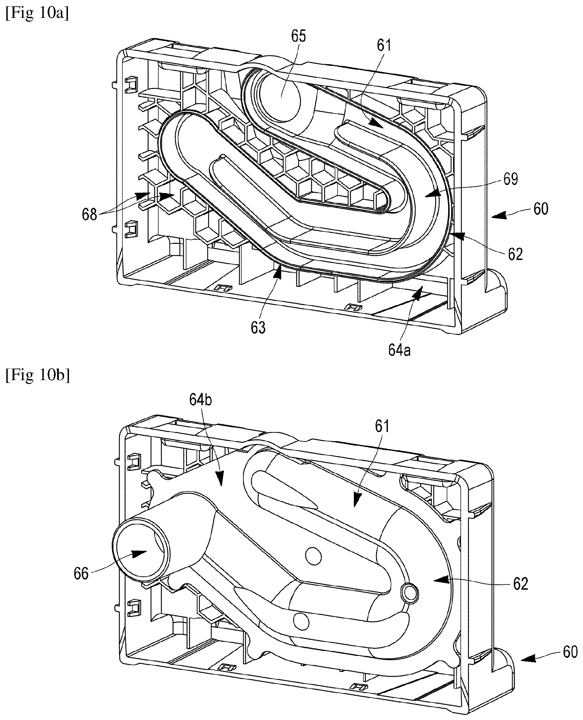

Enfin, avant de quitter l'appareil, le flux d'air traverse une dernière pièce : il s'agit d'une pièce terminale 60 d'atténuation des vibrations et du bruit, représentée en figures 10a et 10b, comportant de fait une chicane placée dans le trajet de circulation de l'air. Celle-ci prend la forme d'une tubulure 61 constituée à partir de deux parties de ladite pièce 60 fixées l'une à l'autre, un socle 64a et une façade 64b, la tubulure 61 créée entre eux se développant dans un plan frontal d'allure perpendiculaire à la direction du flux d'air en sortie du conduit de sortie 6. La longueur parcourue par le flux dans cette tubulure 61, ainsi que la forme et les dimensions de la section transversale au passage du flux, participent à l'efficacité de l'atténuation des vibrations et du bruit. Une nervure séparatrice 69 dépassant du socle 64a permet la fluidification de l'air et l'atténuation des turbulences lors du guidage du flux d'air dans la tubulure 61, conduisant à une réduction de bruit. La chicane présente plus précisément un premier virage 62 en demi-tour suivi d'un virage plus léger 63 infléchissant la direction de la tubulure 61 de sorte que les bouches respectivement d'entrée 65 et de sortie 66 de ladite tubulure 61, soient presque au même niveau. Ces deux bouches 65 et 66 sont d'allure parallèles, entre elles et à la direction donnée au flux à l'issue de la conduite de sortie 6. Cette dernière y présente un embout 67 de fixation à la bouche d'entrée 65 de la pièce terminale 60.

La face visible du socle 64a est recouverte d'une structure à motifs alvéolaires 68. Cette pièce terminale 60 ajoute de fait un morceau de conduite supplémentaire au circuit aéraulique de l'appareil de l'invention décrit jusqu'ici, qui sert à atténuer le niveau sonore émis en aval du compresseur 10, et particulièrement le bruit ressenti spécifiquement dans le masque porté par le patient sur son visage.

Les solutions techniques au problème initialement posé, à savoir diminuer et/ou supprimer quand c'est possible les bruits et vibrations de l'appareil, sont traitées sans recourir à de la mousse. Toutes les caractéristiques mises en avant auparavant pour l'atténuation acoustiques sont au contraire mises en œuvre par des pièces « rigides » servant habituellement à former les conduites du flux d'air : ces caractéristiques s'incarnent dans les formes données à certains éléments, dans des nervures « anti-turbulences », dans des structures alvéolaires en nids d'abeilles implantées dans certains volumes à combler, etc., dont les dimensions en vue d'atténuer le niveau sonore ont fait l'objet de travaux itératifs pour être correctement établies.

Les exemples de la configuration de l'appareil à PPC qui fait l'objet des figures ne doivent pas être considérés comme exhaustifs de l'invention, qui englobe au contraire par exemple des variantes de forme dans les conduites, les structures alvéolaires, les pièces d'entrée et de sortie, etc.

## Revendications

1. Appareil d'assistance respiratoire de type à pression positive continue pour le traitement des troubles respiratoires du sommeil, comportant un conduit d'entrée d'air (5) acheminant un flux d'air entrant vers un compresseur (10), le flux d'air comprimé en sortie du compresseur (10) étant relié à un conduit de sortie d'air (6) pressurisé à destination d'un masque prévu pour s'appliquer aux voies respiratoires d'un patient, **caractérisé en ce que** le conduit d'entrée d'air (5) débouche dans un compartiment (9) en amont - selon le sens de la circulation du flux d'air - de l'entrée du compresseur (10), et présente à ce niveau des moyens de guidage du flux d'air d'abord vers une paroi de fond dudit compartiment (9) orientée sensiblement perpendiculairement à la direction d'aspiration de l'air dans le compresseur (10), au moins ladite paroi de fond étant au moins partiellement recouverte d'une structure à motifs alvéolaires (91) rigide et indéformable.

2. Appareil d'assistance respiratoire de type à pression positive continue selon la revendication précédente, **caractérisé en ce que** le conduit d'entrée d'air (5) comporte, à l'entrée dudit compartiment (9), une rampe (80) suivie d'une portion spiralée (81) se développant dans les motifs alvéolaires (91) de la paroi de fond, ladite portion spiralée (81) présentant un sabot terminal (83) creux arrondi, centré dans ladite paroi de fond et localisé au droit de l'axe du compresseur (10).

3. Appareil d'assistance respiratoire de type à pression positive continue selon la revendication précédente, **caractérisé en ce que** la portion spiralée (81) est munie, en amont du sabot arrondi (83), d'une nervure séparatrice (82).

4. Appareil d'assistance respiratoire de type à pression positive continue selon l'une des revendications 2 et 3, **caractérisé en ce que** la rampe (80) et la portion spiralée (81) constituent la face supérieure d'un bloc (8) dont le socle comporte une structure à motifs alvéolaires (85).

5. Appareil d'assistance respiratoire de type à pression positive continue selon l'une des revendications 2 à 4, **caractérisé en ce qu'**il comporte un espace tampon (11) prévu en amont du compresseur (10), entre d'une part une paroi de séparation (4) séparant le compartiment (9) du compresseur (10), ladite paroi (4) étant munie d'une ouverture (45) placée au droit du sabot terminal (83) de la portion spiralée (81), et d'autre part ledit compresseur (10).

6. Appareil d'assistance respiratoire de type à pression positive continue selon l'une des revendications 2 à 5, **caractérisé en ce que**, en amont de la rampe (80), le conduit d'entrée d'air (5) comporte deux tronçons (54, 56) d'allure parallèle superposés, un premier tronçon (5) relié à l'entrée d'air de l'appareil et un second tronçon (56) débouchant dans la rampe (80), lesdits tronçons (54, 56) étant reliés par une portion arrondie en U (55).

7. Appareil d'assistance respiratoire de type à pression positive continue selon la revendication précédente, **caractérisé en ce que** le premier tronçon comporte un profil linéarisateur (54a), constitué d'un motif alvéolaire orienté dans le sens de passage du flux.

8. Appareil d'assistance respiratoire de type à pression positive continue selon l'une des revendications 6 et 7, **caractérisé en ce que** le second tronçon (56) comporte une sonde Pitot (7) dont une semelle (70) reposant sur une paroi de séparation des tronçons (54, 56) superposés présente une structure à motifs alvéolaires (71).

9. Appareil d'assistance respiratoire de type à pression positive continue selon l'une des revendications précédentes, **caractérisé en ce que** le conduit de sortie (6) en aval du compresseur (10) est équipé d'une pièce terminale (60) d'atténuation des vibrations et du bruit comportant une chicane de circulation de l'air sous forme d'une tubulure (61) présentant au moins un virage (62, 63) et se développant dans un plan frontal d'allure perpendiculaire à la direction du flux d'air dans la portion finale du conduit de sortie (6), les bouches d'entrée (65) et de sortie (66) de ladite tubulure (61) étant sensiblement parallèle à ladite direction.

10. Appareil d'assistance respiratoire de type à pression positive continue selon la revendication précédente, **caractérisé en ce que** la tubulure (61) de la pièce terminale (60) d'atténuation des vibrations et du bruit est constituée à partir de deux parties de ladite pièce (60) fixées l'une à l'autre, un socle (64a) et une façade (64b) orientés selon ledit plan frontal, et **en ce qu'**au moins la face du socle (64a) sur laquelle la façade (64b) est fixée est recouverte d'une structure à motifs alvéolaires (68).

11. Appareil d'assistance respiratoire de type à pression positive continue selon l'une des revendications précédentes, **caractérisé en ce que** le conduit d'entrée d'air (5) est fermé, à son entrée, par un capot porte - filtre (50) muni d'une grille (51) décalée par rapport à l'axe du conduit d'entrée d'air (5), ledit capot (50) étant solidarisé à un pavillon (30) s'évasant à partir du conduit d'entrée (5) et acheminant l'air depuis la grille (51) audit conduit (5).

12. Appareil d'assistance respiratoire de type à pression positive continue selon la revendication précédente, **caractérisé en ce que** des parois latérales d'allure perpendiculaire à la surface de la grille (51) au niveau du capot ou du pavillon comportent des lames obliques (53) inclinées, dépassant desdites parois latérales en direction du centre de la grille (51).

13. Appareil d'assistance respiratoire de type à pression positive continue selon la revendication précédente, **caractérisé en ce que** le capot muni de la grille (51) présente, sur sa face interne en face du conduit d'entrée (5), une structure à motifs alvéolaires.

14. Appareil d'assistance respiratoire de type à pression positive continue selon l'une des revendications précédentes, **caractérisé en ce que** chaque structure à motifs alvéolaires (91, 71, 85, 52, 68) comporte des alvéoles hexagonales en nid d'abeille.

15. Appareil d'assistance respiratoire de type à pression positive continue selon l'une des revendications précédentes, **caractérisé en ce que** le compresseur (10) est fixé dans un châssis (1) comportant deux demi-coques (3, 4) superposées séparées par un joint médian souple (4), les conduits d'entrée (5) et de sortie (6) étant au moins partiellement conformés dans ledit châssis (1).

16. Appareil d'assistance respiratoire de type à pression positive continue selon la revendication précédente, **caractérisé en ce qu'**une sonde Pitot et un bloc (8) à rampe (80) et portion spiralée (81) du conduit d'entrée (5) sont immobilisés dans ledit châssis (1), entre le joint médian souple (4) et les demi-coques (2, 3), le joint médian souple (4) formant une paroi de séparation d'une part entre deux tronçons (54, 56) superposés du conduit d'entrée (5) et d'autre part entre le compresseur (10) et un compartiment (9) au débouché de la rampe (80), ledit joint (4) comportant une ouverture (45) placée au-dessus de l'extrémité de ladite portion spiralée (81) et sous l'entrée du compresseur (10).

## Patentansprüche

1. Atemunterstützungsgerät vom Typ mit kontinuierlichem Überdruck zur Behandlung von Schlafatemstörungen, umfassend eine Lufteinlassleitung (5), die einen einströmenden Luftstrom zu einem Kompressor (10) leitet, wobei der aus dem Kompressor (10) austretende Druckluftstrom mit einer unter Druck stehenden Luftauslassleitung (6) verbunden ist, die zu einer Maske führt, die zur Anwendung an den Atemwegen eines Patienten vorgesehen ist, **dadurch gekennzeichnet, dass** die Lufteinlassleitung (5) in eine Kammer (9) mündet, die - in Strömungsrichtung des Luftstroms - stromaufwärts des Einlasses des Kompressors (10) mündet und an dieser Stelle Mittel aufweist, um den Luftstrom zunächst zu einer Bodenwand der Kammer (9) zu leiten, die im Wesentlichen senkrecht zur Ansaugrichtung der Luft im Kompressor (10) ausgerichtet ist, wobei wenigstens die genannte Bodenwand wenigstens teilweise mit einer starren und unverformbaren Struktur mit Wabenmuster (91) bedeckt ist.

2. Atemunterstützungsgerät vom Typ mit kontinuierlichem Überdruck nach dem vorhergehenden Anspruch, **dadurch gekennzeichnet, dass** die Lufteinlassleitung (5) am Eingang der Kammer (9) eine Rampe (80) aufweist, auf die ein spiralförmiger Abschnitt (81) folgt, der sich in die Wabenmuster (91) der Bodenwand einfügt, wobei der spiralförmige Abschnitt (81) einen hohlen, abgerundeten Endschuh (83) aufweist, der in der Bodenwand zentriert und in Höhe der Achse des Kompressors (10) angeordnet ist.

3. Atemunterstützungsgerät vom Typ mit kontinuierlichem Überdruck nach dem vorhergehenden Anspruch, **dadurch gekennzeichnet, dass** der spiralförmige Abschnitt (81) stromaufwärts des abgerundeten Endschuhs (83) mit einer Trennrippe (82) versehen ist.

4. Atemunterstützungsgerät vom Typ mit kontinuierlichem Überdruck nach einem der Ansprüche 2 und 3, **dadurch gekennzeichnet, dass** die Rampe (80) und der spiralförmige Abschnitt (81) die Oberseite eines Blocks (8) bilden, dessen Sockel eine Struktur mit Wabenmustern (85) aufweist.

5. Atemunterstützungsgerät vom Typ mit kontinuierlichem Überdruck nach einem der Ansprüche 2 bis 4, **dadurch gekennzeichnet, dass** es einen Pufferraum (11) aufweist, der stromaufwärts des Kompressors (10) zwischen einerseits einer Trennwand (4), die die Kammer (9) vom Kompressor (10) trennt, wobei die Wand (4) mit einer Öffnung (45) versehen ist, die sich in Höhe des Endstücks (83) des spiralförmigen Abschnitts (81) befindet, und andererseits dem Kompressor (10) vorgesehen ist.

6. Atemunterstützungsgerät vom Typ mit kontinuierlichem Überdruck nach einem der Ansprüche 2 bis 5, **dadurch gekennzeichnet, dass** stromaufwärts der Rampe (80) die Lufteinlassleitung (5) zwei übereinanderliegende, parallel verlaufende Abschnitte (54, 56) umfasst, wobei ein erster Abschnitt (5) mit dem Lufteinlass des Geräts verbunden ist und ein zweiter Abschnitt (56) in die Rampe (80) mündet, wobei die genannten Abschnitte (54, 56) durch einen U-förmig abgerundeten Abschnitt (55) verbunden sind.

7. Atemunterstützungsgerät vom Typ mit kontinuierlichem Überdruck nach dem vorhergehenden Anspruch, **dadurch gekennzeichnet, dass** der erste Abschnitt ein Linearisierungsprofil (54a) aufweist, das aus einem in Durchflussrichtung ausgerichteten Wabenmuster besteht.

8. Atemunterstützungsgerät vom Typ mit kontinuierlichem Überdruck nach einem der Ansprüche 6 und 7, **dadurch gekennzeichnet, dass** der zweite Abschnitt (56) eine Pitot-Sonde (7) umfasst, deren Sohle (70), die auf einer Trennwand der übereinanderliegenden Abschnitte (54, 56) aufliegt, eine Struktur mit Wabenmustern (71) aufweist.

9. Atemunterstützungsgerät vom Typ mit kontinuierlichem Überdruck nach einem der vorhergehenden Ansprüche, **dadurch gekennzeichnet, dass** die Auslassleitung (6) stromabwärts des Kompressors (10) mit einem Endstück (60) zur Dämpfung von Vibrationen und Geräuschen ausgestattet ist, das eine Luftströmungsbarriere in Form eines Rohrstücks (61) aufweist, die wenigstens eine Biegung (62, 63) aufweist und sich in einer Frontalebene senkrecht zur Richtung des Luftstroms im Endabschnitt der Auslassleitung (6) erstreckt, wobei die Einlass- (65) und Auslassöffnungen (66) des Rohrstücks (61) im Wesentlichen parallel zu dieser Richtung verlaufen.

10. Atemunterstützungsgerät vom Typ mit kontinuierlichem Überdruck nach dem vorhergehenden Anspruch, **dadurch gekennzeichnet, dass** der Schlauch (61) des Endstücks (60) zur Dämpfung von Vibrationen und Geräuschen aus zwei miteinander verbundenen Teilen des Endstücks (60) besteht, einem Sockel (64a) und einer Frontplatte (64b), die in Richtung der Frontalebene ausgerichtet sind, und dadurch, dass wenigstens die Seite des Sockels (64a), an der die Frontplatte (64b) befestigt ist, mit einer Struktur mit Wabenmuster (68) bedeckt ist.

11. Atemunterstützungsgerät vom Typ mit kontinuierlichem Überdruck nach einem der vorhergehenden Ansprüche, **dadurch gekennzeichnet, dass** die Lufteinlassleitung (5) an ihrem Eingang durch eine Filterabdeckung (50) verschlossen ist, die mit einem gegenüber der Achse der Lufteinlassleitung (5) versetzten Gitter (51) versehen ist, wobei die Abdeckung (50) mit einem Trichter (30) verbunden ist, der sich von der Einlassleitung (5) aus erweitert und die Luft vom Gitter (51) zur Leitung (5) leitet.

12. Atemunterstützungsgerät vom Typ mit kontinuierlichem Überdruck nach dem vorhergehenden Anspruch, **dadurch gekennzeichnet, dass** die Seitenwände, die senkrecht zur Oberfläche des Gitters (51) auf Höhe der Abdeckung oder des Trichters verlaufen, schräge Lamellen (53) aufweisen, die von den Seitenwänden in Richtung der Mitte des Gitters (51) überstehen.

13. Atemunterstützungsgerät vom Typ mit kontinuierlichem Überdruck nach dem vorhergehenden Anspruch, **dadurch gekennzeichnet, dass** die mit dem Gitter (51) versehene Abdeckung auf ihrer Innenseite gegenüber der Einlassleitung (5) eine Struktur mit Wabenmustern aufweist.

14. Atemunterstützungsgerät vom Typ mit kontinuierlichem Überdruck nach einem der vorhergehenden Ansprüche, **dadurch gekennzeichnet, dass** jede Struktur mit Wabenmuster (91, 71, 85, 52, 68) sechseckige Waben aufweist.

15. Atemunterstützungsgerät vom Typ mit kontinuierlichem Überdruck nach einem der vorhergehenden Ansprüche, **dadurch gekennzeichnet, dass** der Kompressor (10) in einem Rahmen (1) befestigt ist, der zwei übereinanderliegende Halbschalen (3, 4) umfasst, die durch eine flexible Mittelfuge (4) voneinander getrennt sind, wobei die Einlass- (5) und Auslassleitungen (6) wenigstens teilweise in diesem Rahmen (1) ausgebildet sind.

16. Atemunterstützungsgerät vom Typ mit kontinuierlichem Überdruck nach dem vorhergehenden Anspruch, **dadurch gekennzeichnet, dass** eine Pitot-Sonde und ein Block (8) mit einer Rampe (80) und einem spiralförmigen Abschnitt (81) der Einlassleitung (5) in dem genannten Rahmen (1) zwischen der flexiblen Mittelfuge (4) und den Halbschalen (2, 3) befestigt sind, wobei die flexible Mittelfuge (4) eine Trennwand einerseits zwischen zwei übereinanderliegenden Abschnitten (54, 56) der Einlassleitung (5) und andererseits zwischen dem Kompressor (10) und einer Kammer (9) am Ausgang der Rampe (80) bildet, wobei die Fuge (4) eine Öffnung (45) aufweist, die über dem Ende des spiralförmigen Abschnitts (81) und unter dem Einlass des Kompressors (10) angeordnet ist.

## Claims

1. A continuous positive pressure type breathing assistance device for the treatment of sleep-related breathing disorders, comprising an air inlet duct (5) conveying an incoming air flow to a compressor (10), the compressed air flow output from the compressor (10) being connected to a pressurised air outlet duct (6) intended for a mask provided to be applied to the respiratory tract of a patient, **characterised in that** the air inlet duct (5) opens into a compartment (9) upstream - in the direction of circulation of the air flow - of the inlet of the compressor (10), and has, at this level, means for guiding the air flow first towards a bottom wall of said compartment (9) oriented substantially perpendicular to the direction of intake of air into the compressor (10), at least said bottom wall being at least partially covered by a rigid and non-deformable structure with honeycomb patterns (91).

2. The continuous positive pressure type breathing assistance device according to the preceding claim, **characterised in that** the air inlet duct (5) comprises, at the inlet of said compartment (9), a ramp (80) followed by a spiral portion (81) developing in the honeycomb patterns (91) of the bottom wall, said spiral portion (81) having a rounded hollow terminal shoe (83), centred in said bottom wall and located in line with the axis of the compressor (10).

3. The continuous positive pressure type breathing assistance device according to the preceding claim, **characterised in that** the spiral portion (81) is provided, upstream of the rounded shoe (83), with a separating rib (82).

4. The continuous positive pressure type breathing assistance device according to claims 2 and 3, **characterised in that** the ramp (80) and the spiral portion (81) form the upper surface of a block (8), the base of which comprises a structure with honeycomb patterns (85).

5. The continuous positive pressure type breathing assistance device according to one of claims 2 to 4, **characterised in that** it comprises a buffer space (11) provided upstream of the compressor (10), between, on one hand, a partition wall (4) separating the compartment (9) from the compressor (10), said wall (4) being provided with an opening (45) placed in line with the terminal shoe (83) of the spiral portion (81), and, on the other, said compressor (10).

6. The continuous positive pressure type breathing assistance device according to one of claims 2 to 5, **characterised in that**, upstream of the ramp (80), the air inlet duct (5) comprises two superimposed parallel type sections (54, 56), a first section (5) connected to the air inlet of the device and a second section (56) opening into the ramp (80), said sections (54, 56) being connected by a rounded U-shaped portion (55).

7. The continuous positive pressure type breathing assistance device according to the preceding claim, **characterised in that** the first section comprises a linearising profile (54a), consisting of a honeycomb pattern oriented in the direction of the flow.

8. The continuous positive pressure type breathing assistance device according to one of claims 6 and 7, **characterised in that** the second section (56) comprises a Pitot probe (7), a base plate (70) of which resting on a partition wall between the superimposed sections (54, 56) has a structure with honeycomb patterns (71).

9. The continuous positive pressure type breathing assistance device according to one of the preceding claims, **characterised in that** the outlet duct (6) downstream of the compressor (10) is equipped with an end piece (60) for attenuating vibrations and noise comprising an air circulation baffle in the form of a tube (61) having at least one bend (62, 63) and developing in a frontal type plane perpendicular to the direction of the air flow in the final portion of the outlet duct (6), the inlet (65) and outlet (66) openings of said tube (61) being substantially parallel to said direction.

10. The continuous positive pressure type breathing assistance device according to the preceding claim, **characterised in that** the tube (61) of the end piece (60) for attenuating vibrations and noise is formed from two parts of said piece (60) attached to each other, a base (64a) and a front (64b) oriented along said frontal plane, and **in that** at least the face of the base (64a) on which the front (64b) is attached is covered with a structure with honeycomb patterns (68).

11. The continuous positive pressure type breathing assistance device according to one of the preceding claims, **characterised in that** the air inlet duct (5) is closed, at its inlet, by a filter-holder cover (50) provided with a grille (51) offset relative to the axis of the air inlet duct (5), said cover (50) being secured to a housing (30) flaring from the air inlet duct (5) and conveying air from the grille (51) to said duct (5).

12. The continuous positive pressure type breathing assistance device according to the preceding claim, **characterised in that** the lateral walls of perpendicular type to the surface of the grille (51) at the cover or housing comprise inclined oblique blades (53), protruding from said lateral walls towards the centre of the grille (51).

13. The continuous positive pressure type breathing assistance device according to the preceding claim, **characterised in that** the cover provided with the grille (51) has, on its inner face facing the inlet duct (5), a structure with honeycomb patterns.

14. The continuous positive pressure type breathing assistance device according to one of the preceding claims, **characterised in that** each structure with honeycomb patterns (91, 71, 85, 52, 68) comprises hexagonal honeycomb cells.

15. The continuous positive pressure type breathing assistance device according to one of the preceding claims, **characterised in that** the compressor (10) is attached in a frame (1) comprising two superimposed half-shells (3, 4) separated by a flexible middle seal (4), the inlet (5) and outlet (6) ducts being at least partially shaped in said frame (1).

16. The continuous positive pressure type breathing assistance device according to the preceding claim, **characterised in that** a Pitot probe and a block (8) with a ramp (80) and a spiral portion (81) of the inlet duct (5) are immobilised in said frame (1), between the flexible middle seal (4) and the half-shells (2, 3), the flexible middle seal (4) forming a partition wall, on one hand, between two superimposed sections (54, 56) of the inlet duct (5), and, on the other, between the compressor (10) and a compartment (9) at the outlet of the ramp (80), said seal (4) comprising an opening (45) placed above the end of said spiral portion (81) and under the inlet of the compressor (10).
